# FASCICULE DE BREVET EUROPEEN

(11) **EP 2 328 859 B1**
(45) Date de publication et mention de la délivrance du brevet: **09.05.2012**
(21) Numéro de dépôt: 09782452.8
(22) Date de dépôt: 01.09.2009
(51) Int. Cl.: C07C 209/48, C07C 211/12, B01J 25/02

(54) **PROCÉDÉ DE FABRICATION D'AMINES**
VERFAHREN ZUR HERSTELLUNG VON AMINEN
METHOD FOR PRODUCING AMINES

(30) Priorité: 09.09.2008 FR 0804935
(43) Date de publication de la demande: 08.06.2011
(73) Titulaire: Rhodia Opérations, 93306 Aubervilliers (FR)
(72) Inventeur: LETOURNEUR, Didier, F-68170 Rixheim (FR); VERDIER, Stephan, F-69008 Lyon (FR)
(74) Mandataire: Boittiaux, Vincent
(86) Numéro de dépôt international: PCT/EP2009/061268
(87) Numéro de publication internationale: WO 2010/028982

(56) Documents cités:
- WO-A-03/062188
- FR-A- 2 722 709
- FR-A- 2 905 948
- FREIDLIN L KH ET AL: "CATALYTIC REDUCTION OF DINITRILES" RUSSIAN CHEMICAL REVIEWS (USPEKHI KHIMII), INSTITUTE OF PHYSICS PUBLISHING, BRISTOL, GB, vol. 33, no. 6, 1 juin 1964 (1964-06-01), page 319, XP008063249 ISSN: 0036-021X cité dans la demande

## Description

La présente invention concerne un procédé de fabrication de composés amines, plus particulièrement de composés diamines par hydrogénation de composés comprenant des fonctions nitriles.

Elle se rapporte plus particulièrement à la fabrication d'hexaméthylènediamine par hydrogénation du tétraméthylène dicyanure.

L'hexaméthylène diamine est un composé utilisé dans de nombreuses applications dont les principales sont la fabrication de polyamides tels que le polyhexamethylène adipamide, et la fabrication de diisocyanates.

Plusieurs procédés de fabrication de l'hexaméthylène diamine ont été proposés qui consistent généralement en une hydrogénation d'un composé comprenant des fonctions nitriles tels que le tétraméthylène dicyanure en présence d'un catalyseur d'hydrogénation. Deux types de procédé sont exploités industriellement utilisant des catalyseurs et des conditions de température et pression différentes.

Ainsi, un premier type de procédé d'hydrogénation exploité et décrit dans la littérature consiste à hydrogéner les composés nitriles en présence d'ammoniac et sous haute pression, avec un catalyseur à base de ruthénium, par exemple.

Un second type de procédé consiste à réaliser l'hydrogénation des composés nitriles sous une pression proche de la pression atmosphérique et à une température peu élevée, en présence d'un composé basique et d'un catalyseur à base de nickel de Raney.

Dans ce dernier type de procédé, l'hydrogénation des composés nitriles en amines s'opère en présence d'un catalyseur à base de nickel de Raney, éventuellement dopé. Ces catalyseurs sont préparés par lixiviation de l'aluminium, d'alliages Ni-Al riches en aluminium, en milieu fortement alcalin. Les catalyseurs obtenus sont constitués par des agglomérats de cristallites de nickel, ayant une importante surface spécifique et une teneur résiduelle en aluminium variable.

La modification des facteurs structuraux et électroniques du nickel de Raney, par l'addition de métaux à l'alliage nickel-aluminium, a déjà été envisagée. Classiquement, l'addition d'un dopant s'effectue par introduction de ce dopant dans un alliage précurseur Ni-Al en fusion. Il s'agit du dopage métallurgique. Ainsi, le dopage de nickel de Raney par divers promoteurs métalliques (Fe, Co, Cr, Mn, V, Mo, Zr, Ta, Ti), ainsi que leurs incidences quant à l'activité, la sélectivité et la stabilité du catalyseur, font l'objet d'une riche littérature scientifique et technique.

L'article de FREIDLIN et al (Russian Chemical Review, vol. 33, n° 6, june 1964) traite de la réduction catalytique de dinitriles et fait l'inventaire d'un certain nombre de catalyseurs de Raney dopés, mis en oeuvre dans des conditions d'hydrogénation (température, pression en hydrogène (PH₂), milieu réactionnel) variées. Des catalyseurs à base de nickel de Raney dopé au chrome, au cuivre ou au titane sont notamment cités.

Le brevet FR-A 2 068 953 concerne des catalyseurs Ni de Raney dopés au chrome par voie métallurgique.

Les brevets FR-A 2 722 709 et FR-A 2 905 948 ont trait à un catalyseur d'hydrogénation de nitriles en amines, de type Nickel de Raney dopé par au moins un élément choisi parmi les groupes IIB, IVB à VIIB de la classification périodique.

Toutefois, l'utilisation d'un nickel de Raney dopé au chrome ne permet pas d'atteindre des teneurs négligeables en impuretés dans le milieu d'hydrogénation de nitriles comme par exemple une faible concentration en diaminocyclohexane (DCH). Or, ces impuretés comme le DCH, sont particulièrement gênantes car elles ont sensiblement la même température d'ébullition que les amines visées et sont donc très difficiles à séparer.

Il existe donc un besoin industriel d'optimisation des conditions d'hydrogénation de nitriles en amines, notamment des dinitriles en aminonitriles et/ou diamines, au moyen de catalyseurs de type Ni de Raney dopés, notamment au regard des conditions opératoires, ainsi que de l'activité, la sélectivité et la stabilité du catalyseur final.

Une telle optimisation constitue l'un des objets essentiels de la présente invention, qui consiste en un procédé d'hydrogénation de nitriles en amines facile à mettre en oeuvre, non polluant, économique et permettant, d'une part, d'atteindre des sélectivités en amine supérieures à 90% exprimées par rapport au substrat nitrile de départ et, d'autre part, de réduire au maximum la formation d'impuretés, notamment la formation du DCH.

L'invention a notamment pour objet un procédé de fabrication de composés diamines par hydrogénation de composés comprenant des fonctions nitriles tels que des composés dinitriles par réaction avec de l'hydrogène ou un gaz contenant de l'hydrogène moléculaire en présence d'un catalyseur d'hydrogénation à base de nickel de Raney caractérisé en ce que le catalyseur comprend du nickel de Raney et comme éléments métalliques dopants du fer, du chrome et du zinc.

Selon une caractéristique préférentielle de l'invention, la composition pondérale du catalyseur exprimée en masse d'élément métallique est la suivante :
Concentration en poids de fer comprise entre 0,3% et 3%
Concentration en poids de chrome comprise entre 0,5% et 5%
Concentration en poids de zinc comprise entre 0,5% et 5%
Concentration en poids d'aluminium comprise entre 2% et 10%
Le complément à 100% est du nickel

Encore plus avantageusement, la concentration pondérale du catalyseur selon l'invention est la suivante :
Concentration en poids de fer comprise entre 1 % et 2%
Concentration en poids de chrome comprise entre 1,5% et 2,5%
Concentration en poids de zinc comprise entre 1% et 3%
Concentration en poids d'aluminium comprise entre 5% et 7%
Le complément à 100% est du nickel

Le catalyseur Ni de Raney dopé mis en oeuvre dans ce procédé provient généralement d'un alliage précurseur Ni-A1 (teneur en Ni de 28 à 59% en poids), mis en fusion et additionné des éléments métalliques dopants : le fer, le chrome et le zinc, selon une procédure de dopage dite « métallurgique ». Après refroidissement, l'alliage précurseur dopé est soumis, de manière traditionnelle, à une attaque alcaline provoquant une élimination plus ou moins importante de l'aluminium et, éventuellement, d'une fraction de l'élément dopant.

Les alliages de départ mis en oeuvre sont, avantageusement, choisis parmi les formes suivantes d'associations binaires nickel/aluminium: NiAl₃, Ni₂Al₃, et proeutectique Al/NiAl₃.

Le dopage du catalyseur Ni de Raney peut être également réalisé par voie chimique. Ainsi, au moins un des dopants peut être ajouté à la composition par ce mode de dopage. Il est également possible d'introduire la totalité des dopants par ce dopage dit « chimique ».

Selon un premier mode de réalisation de dopage chimique, le catalyseur Ni de Raney est imprégné avec une solution contenant un précurseur de l'élément dopant. Les différents modes d'imprégnation usuels, connus par l'homme du métier sont possibles. L'imprégnation consiste à mélanger le catalyseur avec une solution du précurseur du dopant. La majeure partie du solvant est évaporé puis le catalyseur est traité thermiquement et éventuellement lavé à l'eau.

Selon un second mode de réalisation du procédé de dopage chimique, l'élément dopant est précipité sur le catalyseur Ni de Raney. Dans ce cas, le catalyseur Ni de Raney est mis en suspension dans un solvant. Un composé, précurseur du dopant est ajouté à la suspension puis précipité sur le catalyseur. En fonction de la nature du précurseur, La précipitation est obtenue par, par exemple, modification du pH de la suspension par ajout d'une base ou par augmentation de la température du milieu pendant une durée variant de 0,5 à 10h à une température comprise entre la température ambiante (environ 20-25°C) et le point d'ébullition du solvant utilisé. Après décantation du catalyseur, le liquide surnageant est extrait et le catalyseur est lavé à l'eau.

Dans ces deux modes de réalisation de dopage chimique, le solvant utilisé est, de préférence, de l'eau. L'utilisation de solvants organiques est possible en fonction de la solubilité du composé précurseur du dopant. Comme précurseurs du dopant, on peut utiliser des composés organiques ou inorganiques. Comme exemples de composés organiques, on peut citer les carboxylates ou les alcoolates métalliques. Comme exemples de composés inorganiques, on peut citer les chlorures, les nitrates, les sulfates, les hydroxydes ou les oxydes. Comme base, on peut utiliser différents composés basiques tels que les amines ou les carbonates et hydroxydes alcalins ou alcalino-terreux. On utilise de préférence la soude ou la potasse.

Enfin, selon un dernier mode de réalisation, le composé précurseur du dopant peut être introduit pendant l'attaque alcaline de l'alliage de Raney. L'alliage de Raney est mis en suspension dans une solution aqueuse de soude pour obtenir un pH supérieur à 10, de préférence supérieur à 12. Le composé précurseur du dopant est ajouté à la suspension et la température est ajustée entre 90 et 100°C pendant une durée de 0,5h à 10h. Après décantation ou sédimentation du catalyseur, le liquide surnageant est extrait et le catalyseur est lavé à l'eau.

Le procédé d'hydrogénation de l'invention s'applique, plus particulièrement mais non limitativement, aux composés nitriles de formule (I) :

NC-R-CN (I)

Dans laquelle R représente un groupement alkylène ou alcénylène, linéaire ou ramifié, ayant de 1 à 12 atomes de carbone, ou un groupement arylène ou aralkylène ou aralcénylène substitué ou non.

De préférence, on met en oeuvre dans le procédé de l'invention des composés dinitriles de formule (I) dans laquelle R représente un radical alkylène, linéaire ou ramifié ayant de 2 à 6 atomes de carbone.

A titre d'exemples de tels composés dinitriles, on peut citer notamment l'adiponitrile ou tétraméthylène dicyanure, le méthylglutaronitrile, l'éthylsuccinonitrile, le malononitrile, le succinonitrile et le glutaronitrile et leurs mélanges, notamment les mélanges adiponitrile, méthylglutaronitrile, éthylsuccinonitrile qui proviennent d'un même procédé de synthèse de l'adiponitrile.

L'introduction du substrat nitrile, par exemple l'adiponitrile, dans le milieu réactionnel se fait en respectant une concentration comprise entre 0,001 % et 30 % en poids par rapport au poids total (p/p) du milieu réactionnel et de préférence entre 0,1 % et 20 % p/p.

De façon privilégiée, la base forte mise en oeuvre est choisie parmi les composés suivants : LiOH, NaOH, KOH, RbOH, CsOH et leur mélanges.

En pratique, on utilise préférentiellement NaOH et KOH, pour un bon compromis performance-prix.

Le milieu réactionnel d'hydrogénation est de préférence liquide. Il peut contenir un solvant apte à solubiliser le substrat nitrile à hydrogéner, sachant que cette transformation s'opère mieux lorsque ledit substrat se trouve en solution.

Suivant un mode préférentiel du procédé de l'invention, on utilise un milieu réactionnel liquide au moins partiellement aqueux sans solvant. L'eau est généralement présente dans une quantité inférieure ou égale à 50 % en poids, avantageusement inférieure ou égale à 20 % en poids par rapport au milieu réactionnel total. Plus préférentiellement encore, la teneur en eau du milieu réactionnel est comprise entre 0,1 et 15 % en poids par rapport à l'ensemble des constituants dudit milieu.

En complément ou en substitution à l'eau, on peut éventuellement prévoir au moins un autre solvant, du type alcool et/ou amide. Les alcools qui conviennent plus particulièrement sont par exemple le méthanol, l'éthanol, le propanol, l'isopropanol, le butanol, les glycols, tels que l'éthylène et/ou le propylène glycol, des polyols et/ou des mélanges desdits composés.

Dans le cas où le solvant est constitué par un amide, il peut s'agir par exemple du diméthylformamide ou du diméthylacétamide.

Lorsqu'il est employé avec l'eau, le solvant, de préférence alcoolique, représente de deux à quatre parties en poids pour une partie en poids d'eau et de préférence trois parties pour une partie d'eau.

Selon une autre caractéristique préférée de l'invention, on incorpore de l'amine, dont la préparation est visée par le procédé, au sein du milieu réactionnel. Il s'agit par exemple d'hexaméthylènediamine, lorsque le substrat nitrile est l'adiponitrile.

La concentration de l'amine visée dans le milieu réactionnel est avantageusement comprise entre 50 % et 99 % en poids par rapport à la totalité du milieu réactionnel et, plus préférentiellement encore, est comprise entre 60 % et 99 % en poids.

La quantité de base dans le milieu réactionnel varie en fonction de la nature du milieu réactionnel.

Dès lors que le milieu réactionnel ne contient que de l'eau et de l'amine visée, à titre de milieu solvant liquide, la quantité de base est avantageusement supérieure ou égale à 0,1 mol/kg de catalyseur, de préférence comprise entre 0,1 et 2 mol/kg de catalyseur et plus préférentiellement encore entre 0,5 et 1,5 mol/kg de catalyseur.

Dans le cas où le milieu réactionnel comprend de l'eau et un alcool et/ou un amide, la quantité de base est supérieure ou égale à 0,05 mol/kg de catalyseur, est comprise de préférence entre 0,1 et 10,0 mol/kg et plus préférentiellement encore entre 1,0 et 8,0 mol/kg.

Une fois arrêtée la composition du milieu réactionnel et le choix du catalyseur, on procède à un mélange de ces deux éléments, puis on chauffe ce mélange à une température réactionnelle inférieure ou égale à 150°C, de préférence inférieure ou égale à 120°C et, plus préférentiellement encore, inférieure ou égale à 100°C.

Concrètement, cette température est comprise entre la température ambiante (20°C environ) et 100°C.

Préalablement, simultanément ou postérieurement au chauffage, l'enceinte réactionnelle est amenée à la pression en hydrogène convenable, c'est-à-dire, en pratique, comprise entre 0,10 et 10 MPa.

La durée de la réaction est variable en fonction des conditions réactionnelles et du catalyseur.

Dans un mode de fonctionnement discontinu, elle peut varier de quelques minutes à plusieurs heures.

Dans un mode de fonctionnement continu, qui est parfaitement envisageable pour le procédé selon l'invention, la durée n'est évidemment pas un paramètre figeable.

Il est à noter que l'homme du métier peut modifier la chronologie des étapes du procédé selon l'invention, selon les conditions opératoires. L'ordre donné ci-avant ne correspond qu'à une forme préférée, mais non limitative, du procédé selon l'invention.

Les autres conditions qui régissent l'hydrogénation (en mode continu ou discontinu) conforme à l'invention, relèvent de dispositions techniques traditionnelles et connues en elles-mêmes.

Grâce à toutes les dispositions avantageuses évoquées ci-dessus, le procédé de l'invention permet d'hydrogéner des substrats nitriles en amines, de façon sélective, rapide, commode et économique, avec une formation d'impuretés, notamment de DCH très inférieure à celle obtenue avec un catalyseur Nickel de Raney dopé par du chrome ou par du fer et du chrome.

Ce procédé est parfaitement adapté pour transformer l'adiponitrile en hexaméthylènediamine, monomère utilisé notamment dans la synthèse du polyamide-6,6.

L'invention sera mieux comprise et ses avantages et variantes de mise en oeuvre ressortiront bien des exemples qui suivent illustrant, de façon non limitative, le procédé d'hydrogénation selon l'invention

### Exemple 1 :

Dans cet exemple, on décrit la préparation d'un catalyseur Ni de Raney dopé par du fer et du chrome. Ce catalyseur sera utilisé comme catalyseur de référence.

### -Fabrication de l'alliage :

Dans un creuset 75 kg d'aluminium en lingots sont fondus à une température d'environ 800°C. 75 kg de nickel et 2,7 kg d'un alliage fer-chrome sont ajoutés dans le creuset et la température est portée à 1450°C. Le milieu est homogénéisé. L'alliage en fusion obtenu est coulé en lingotières, refroidi, démoulé, concassé et broyé pour obtenir une poudre.

La composition massique de l'alliage est la suivante :

| | |
|---|---|
| Ni: | 47,80 % |
| Al : | 50,15 % |
| Fe : | 0,85 % |
| Cr : | 1,20 % |

### - Attaque alcaline :

Dans un réacteur muni d'une agitation, 0,5 L de soude aqueuse à 30 % en poids sont introduits. La température du milieu est portée à 97°C et le ciel du réacteur est balayé avec de l'argon. 50 g poudre d'alliage sont progressivement introduits dans le réacteur toujours sous balayage d'argon. L'addition d'alliage terminée, l'attaque alcaline est poursuivie pendant 3h. Après sédimentation du catalyseur, le liquide surnageant est extrait. Le catalyseur est lavé avec de l'eau jusqu'à l'obtention d'un pH de 7 puis stocké dans une solution de soude à 0,05 mol/L.

La composition massique du catalyseur obtenu est la suivante :

| | |
|---|---|
| Ni : | 88,1% |
| Al : | 8,0% |
| Fe : | 1,5% |
| Cr : | 2,4% |

Soit des rapports massiques :

| | |
|---|---|
| Al/Ni : | 9,0 % |
| Fe/Ni : | 1,7 % |
| Cr/Ni : | 2,7 % |

### Exemple 2 :

Dans cet exemple, on décrit la préparation d'un catalyseur Ni de Raney dopé par du fer, du chrome et du zinc.

Dans un réacteur muni d'une agitation, 415 mL d'une solution de soude à 20% poids et 1,0 g d'oxyde de zinc sont introduits. La température du milieu est portée à 90°C et le ciel du réacteur est balayé avec de l'argon. 81 g d'alliage obtenu selon l'exemple 1 sont introduits dans le réacteur avec un débit de 0,7 g/min. L'addition d'alliage terminée, l'attaque alcaline est poursuivie pendant 3h à une température de 98°C. Après sédimentation du catalyseur, le liquide surnageant est extrait. Le catalyseur est lavé avec de l'eau jusqu'à obtention d'un pH de 7 puis stocké dans une solution de soude à 0,05 mol/L.

La composition massique du catalyseur obtenu est la suivante :

| | |
|---|---|
| Ni: | 87,80 % |
| Al : | 8,00 % |
| Fe: | 1,05% |
| Cr : | 2,15% |
| Zn : | 1,00 % |

Soit des rapports massique :

| | |
|---|---|
| Al/Ni : | 9,10 % |
| Fe/Ni : | 1,20 % |
| Cr/Ni : | 2,45% |
| Zn/Ni : | 1,15 % |

### Exemple 3 :

Dans cet exemple, on décrit la préparation d'un catalyseur Ni de Raney dopé par du fer, du chrome et du zinc.

Dans un réacteur muni d'une agitation, 415 mL d'une solution de soude à 20% poids et 1,67 g de chlorure de zinc anhydre sont introduits. La température du milieu est portée à 90°C et le ciel du réacteur est balayé avec de l'argon. 80 g d'alliage obtenu selon l'exemple 1 sont introduits dans le réacteur avec un débit de 0,7 g/min. L'addition d'alliage terminée, l'attaque alcaline est poursuivie pendant 3h à une température de 98°C. Après sédimentation du catalyseur, le liquide surnageant est extrait. Le catalyseur est lavé avec de l'eau jusqu'à obtention d'un pH de 7 puis stocké dans une solution de soude à 0,05 mol/L.

La composition massique du catalyseur obtenu est la suivante :

| | |
|---|---|
| Ni: | 87,95 % |
| Al : | 8,30 % |
| Fe : | 1,10 % |
| Cr: | 2,10 % |
| Zn: | 1,05% |
| CI: | <10 ppm |

Soit des rapports massiques :

| | |
|---|---|
| Al/Ni : | 9,40 % |
| Fe/Ni: | 1,25 % |
| Cr/Ni : | 2,40 % |
| Zn/Ni : | 1,20 % |

### Exemple 4

On décrit dans cet exemple le test catalytique d'hydrogénation de l'adiponitrile (AdN) en hexaméthylènediamine (HMD) par l'hydrogène en présence du catalyseur Ni de Raney selon l'exemple 1.

La réaction d'hydrogénation est effectuée dans un réacteur inox de 300 mL équipé de :
- d'une sonde de température
- d'une turbine de Rushton auto-aspirante
- d'un régulateur de pression qui permet de maintenir la pression constante dans le réacteur
- d'une pompe qui permet d'introduire en continu l'AdN
- d'un pousse-seringue qui permet d'introduire en continu la solution aqueuse de potasse (KOH)
- d'une vanne d'échantillonnage séquencé qui permet de prélever le milieu réactionnel

Dans ce réacteur, on introduit au démarrage :
- 114,5 g d'HMD et 16,5 g d'eau déminéralisée
- 3,5 g de catalyseur obtenu selon l'exemple 1
- 0,42 g d'une solution de potasse 6,8 mol/L

La température du réacteur est fixée à 80°C. La turbine est mise en route à 1300 tours/min. Le réacteur est purgé à l'azote avant d'être mis sous 25 bar de pression d'hydrogène.

A l'instant t=0, les dispositifs d'injection et prélèvement en continu sont mis en route :
- injection d'AdN dans le réacteur avec un débit de 0,45 mL/min
- injection de solution de potasse 400 ppm avec un débit de 0,05 mL/min
- prélèvement de 0,63 mL par la vanne d'échantillonnage toutes les minutes

L'hydrogène est alimenté en continu dans le réacteur par l'intermédiaire du régulateur de pression. La consommation d'hydrogène est suivie dans le temps par contrôle de la perte de pression dans le réservoir d'alimentation en hydrogène ce qui permet de mesurer l'activité du catalyseur.

Le milieu réactionnel prélevé est collecté dans un flacon sous gaz inerte thermostaté à 60°C pendant les 3h de test.

Les échantillons prélevés périodiquement au cours du test sont analysés en chromatographie en phase gazeuse (CPG) afin de suivre la formation des impuretés formées lors de l'hydrogénation de l'AdN en HMD, plus particulièrement les deux impuretés suivantes :
- le diaminocylohexane (DCH)
- la bishexaméthylènetriamine (BHT)

L'analyse CPG est effectuée à l'aide d'un chromatographe Hewlett Packard HP 6890 équipé d'une colonne capillaire CAM de 30 m de J&W Scientific. Les conditions expérimentales sont les suivantes :
- gaz vecteur H2
- injecteur à 250°C
- détecteur ionisation de flamme FID à 250°C
- rapport de split 80%
- programmation de température : palier 60°C/4min / rampe 4°C/min jusqu'à 100°C / palier 100°C/5min / rampe 10°C/min jusqu'à 210°C / palier 210°C/2min / rampe 10°C/min jusqu'à 220°C / palier 220°C/27min

L'échantillon pour CPG est préparé en mélangeant :
- environ 2 g pesé précisément de milieu réactionnel
- environ 30 mg pesé précisément de nonylamine (étalon interne)
- et environ 0,5 à 1 mL de méthanol

### Exemple 5

On décrit dans cet exemple le test catalytique d'hydrogénation de l'adiponitrile (AdN) en hexaméthylènediamine (HMD) par l'hydrogène en présence du catalyseur Ni de Raney selon l'exemple 2.

On procède comme dans l'exemple 4 mais en engageant le catalyseur selon l'exemple 2.

### Exemple 6

On décrit dans cet exemple le test catalytique d'hydrogénation de l'adiponitrile (AdN) en hexaméthylènediamine (HMD) par l'hydrogène en présence du catalyseur Ni de Raney selon l'exemple 3.

On procède comme dans l'exemple 4 mais en engageant le catalyseur selon l'exemple 3.

Le Tableau 1 ci-dessous rassemble les résultats obtenus au cours des tests d'hydrogénation des exemples 4, 5 et 6.

| | Exemple 4 | Exemple 5 | Exemple 6 |
|---|---|---|---|
| Catalyseur | Exemple 1 | Exemple 2 | Exemple 3 |
| Activité initiale du catalyseur(10⁻⁵ mol H2/g/s) | 110 | 125 | 120 |
| concentration en DCH (ppm) | 1700 | 1250 | - |
| Concentration en BHT (ppm) | 800 | 475 | - |

Ces essais démontrent clairement que le catalyseur conforme à l'invention présente une activité catalytique équivalente ou supérieure à celle du catalyseur connu et permet d'obtenir une réaction plus sélective en diminuant sensiblement la formation d'impuretés.

## Revendications

1. Procédé de fabrication de composés diamines par hydrogénation de composés comprenant des fonctions nitriles par réaction ave de l'hydrogène ou un gaz contenant de l'hydrogène en présence d'un catalyseur d'hydrogénation à base de nickel de Raney, **caractérisé en ce que** le catalyseur d'hydrogénation comprend du nickel de Raney, du fer, du chrome et du zinc comme éléments dopants.

2. Procédé selon la revendication 1, **caractérisé en ce que** la composition pondérale exprimé en poids d'élément métallique du catalyseur d'hydrogénation est :
- Al : de 2% à 10%
- Fe : de 0,3% à 3%
- Cr : de 0,5% à 5%
- Zn : de 0,5% à 5%
- Ni : complément à 100%

3. Procédé selon la revendication 2, **caractérisé en ce que** la composition pondérale du catalyseur d'hydrogénation est :
- Al : de 5% à 7%
- Fe : de 1% à 2%
- Cr : de 1,5% à 2,5%
- Zn: de 1% à 3%
- Ni : complément à 100%

4. Procédé selon l'une des revendications précédentes, **caractérisé en ce que** le composé comprenant des fonctions nitriles est le tétraméthylène dicyanure.

5. Procédé selon l'une des revendications précédentes, **caractérisé en ce que** le composé diamine est l'hexaméthylène diamine.

6. Procédé selon l'une des revendications précédentes, **caractérisé en ce que** l'hydrogénation est réalisée en présence d'un composé basique.

7. Procédé selon l'une des revendications précédentes, **caractérisé en ce que** la réaction d'hydrogénation est réalisée sous une pression comprise entre 0,1 et 10 MPa

8. Procédé selon l'une des revendications précédentes, **caractérisé en ce que** la réaction d'hydrogénation est réalisée à une température comprise entre 20 et 100 °C.

## Claims

1. Process for manufacturing diamine compounds by hydrogenation of compounds comprising nitrile functional groups by reaction with hydrogen or a gas that contains hydrogen in the presence of a hydrogenation catalyst based on Raney nickel, **characterized in that** the hydrogenation catalyst comprises Raney nickel, iron, chromium and zinc as dopant elements.

2. Process according to Claim 1, **characterized in that** the weight composition, expressed by weight of metallic element, of the hydrogenation catalyst is:
- Al: from 2% to 10%;
- Fe: from 0.3% to 3%;
- Cr: from 0.5% to 5%;
- Zn: from 0.5% to 5%; and
- Ni: balance to 100%.

3. Process according to Claim 2, **characterized in that** the weight composition of the hydrogenation catalyst is:
- Al: from 5% to 7%;
- Fe: from 1% to 2%;
- Cr: from 1.5% to 2.5%;
- Zn: from 1% to 3%; and
- Ni: balance to 100%.

4. Process according to one of the preceding claims, **characterized in that** the compound comprising nitrile functional groups is tetramethylene dicyanide.

5. Process according to one of the preceding claims, **characterized in that** the diamine compound is hexamethylenediamine.

6. Process according to one of the preceding claims, **characterized in that** the hydrogenation is carried out in the presence of a basic compound.

7. Process according to one of the preceding claims, **characterized in that** the hydrogenation reaction is carried out under a pressure between 0.1 and 10 MPa.

8. Process according to one of the preceding claims, **characterized in that** the hydrogenation reaction is carried out at a temperature between 20 and 100°C.

## Patentansprüche

1. Verfahren zur Herstellung von Diaminverbindungen durch Hydrierung von Verbindungen mit Nitrilfunktionen durch Reaktion mit Wasserstoff oder einem Wasserstoff enthaltenden Gas in Gegenwart eines Katalysators auf Basis von Raney-Nickel, **dadurch gekennzeichnet, dass** der Hydrierkatalysator Raney-Nickel, Eisen, Chrom und Zink als Dotierungselemente umfasst.

2. Verfahren nach Anspruch 1, **dadurch gekennzeichnet, dass** die als Gewicht des metallischen Elements ausgedrückte Gewichtszusammensetzung des Hydrierkatalysators wie folgt ist:
- Al: 2% bis 10%
- Fe: 0,3% bis 3%
- Cr: 0,5% bis 5%
- Zn: 0,5% bis 5%
- Ni: Rest auf 100%.

3. Verfahren nach Anspruch 2, **dadurch gekennzeichnet, dass** die Gewichtszusammensetzung des Hydrierkatalysators wie folgt ist:
- Al: 5% bis 7%
- Fe: 1% bis 2%
- Cr: 1,5% bis 2,5%
- Zn: 1% bis 3%
- Ni: Rest auf 100%.

4. Verfahren nach einem der vorhergehenden Ansprüche, **dadurch gekennzeichnet, dass** es sich bei der Verbindung mit Nitrilfunktionen um Tetramethylendicyanid handelt.

5. Verfahren nach einem der vorhergehenden Ansprüche, **dadurch gekennzeichnet, dass** es sich bei der Diaminverbindung um Hexamethylendiamin handelt.

6. Verfahren nach einem der vorhergehenden Ansprüche, **dadurch gekennzeichnet, dass** man die Hydrierung in Gegenwart einer basischen Verbindung durchführt.

7. Verfahren nach einem der vorhergehenden Ansprüche, **dadurch gekennzeichnet, dass** man die Hydrierungsreaktion unter einem Druck zwischen 0,1 und 10 MPa durchführt.

8. Verfahren nach einem der vorhergehenden Ansprüche, **dadurch gekennzeichnet, dass** man die Hydrierungsreaktion bei einer Temperatur zwischen 20 und 100°C durchführt.
